(19) Europäisches Patentamt
European Patent Office
Office européen des brevets

(11) **EP 3 456 706 A1**

(12) **EUROPÄISCHE PATENTANMELDUNG**

(43) Veröffentlichungstag:
**20.03.2019 Patentblatt 2019/12**

(51) Int Cl.:
***C07C 227/40*** (2006.01) ***C07C 229/46*** (2006.01)

(21) Anmeldenummer: **17191793.3**

(22) Anmeldetag: **19.09.2017**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA MD**

(71) Anmelder: **Covestro Deutschland AG**
**51373 Leverkusen (DE)**

(72) Erfinder: **Die Erfindernennung liegt noch nicht vor**

(74) Vertreter: **Levpat**
**c/o Covestro AG**
**Gebäude 4825**
**51365 Leverkusen (DE)**

(54) **POLYASPARAGINSÄUREESTER-ZUSAMMENSETZUNGEN UND VERFAHREN ZUR REINIGUNG**

(57) Die vorliegende Erfindung betrifft ein Verfahren zur Reinigung von Polyasparaginsäureester-Zusammensetzungen und die Bereitstellung von Polyasparaginsäureestern enthaltend 0.01 bis < 2 Gew. -Prozent Fumarsäuredialkylester.

EP 3 456 706 A1

## Beschreibung

Die vorliegende Erfindung betrifft ein Verfahren zur Reinigung von Polyasparaginsäureester-Zusammensetzungen und die Bereitstellung von Polyasparaginsäureestern enthaltend 0.01 bis < 2 Gew. -Prozent Fumarsäuredialkylester.

Polyasparaginsäureester finden Verwendung in Zweikomponenten-Beschichtungsmitteln, die als Bindemittel eine Polyisocyanatkomponente enthalten. Solche Beschichtungsmittel eignen sich zur Herstellung von hochwertigen Überzügen, die hart, elastisch, abrieb- und lösungsmittelbeständig eingestellt werden können. Vorteilhaft gegenüber primären Aminen ist die moderate Reaktivität der in den Polyasparaginsäureestern enthaltenen Amin-Funktionen gegenüber Isocyanaten.

In EP0403921, EP0639628, EP0667362, EP0689881, US5,214,086, US6,605,684, EP0573860, EP0699696, EP0596360, EP0893458, DE19701835, DE102006002153, EP1767559, WO2001007504, WO2001007399, WO2004033517, US6458293, EP1426397 und US5,243,012 werden beispielhaft niedrigviskose Polyasparaginsäureester beschrieben, welche sekundäre Aminogruppen aufweisen und daher eine, verglichen mit primären Aminen, gemäßigtere Reaktivität gegenüber Isocyanaten aufweisen. Solche Polyasparaginsäureester werden auch Aspartate genannt und eignen sich besonders zur Herstellung lösemittelarmer oder -freier Beschichtungsmittel, die eine rasche Aushärtung zeigen.

Die Synthese der Polyasparaginsäureester ist an sich bekannt und erfolgt über eine Addition von primären Aminen an eine aktivierte Kohlenstoff-Doppelbindung vinyloger Carbonylverbindungen, wie beispielsweise in Malein- oder Fumarsäureestern enthalten, die in der Literatur hinreichend beschrieben ist (Hauben Weyl, Meth. d. Org. Chemie Bd. 11/1, 272 (1957), Usp. Khim. 1969, 38, 1933).

Die Umsetzung der Malein- oder Fumarsäureester mit primären Aminen erfolgt unter Verwendung solcher Mengenverhältnisse, dass auf jede primäre Aminogruppe mindestens eine und vorzugsweise genau eine olefinische Doppelbindung entfällt. Während der Herstellung eines Polyasparaginsäureesters auf Basis von Maleinsäure- oder Fumarsäureestern kann eine unerwünschte Nebenreaktion erfolgen, bei der Fumarsäuredialkylester (Dialkylfumarat) als Nebenkomponente gebildet wird. Ein typisches Herstellverfahren eines Polyasparaginsäureesters erfordert eine Lagerzeit von 4-6 Wochen nachdem der Hauptteil der Edukte miteinander reagiert hat. In dieser Zeit erfolgt die sogenannte Reifung des Produktes, welche sich durch Stabilisierung der Viskosität manifestiert. Dadurch, dass der Umsatz innerhalb dieser Zeit weiter steigt, sinkt auch der Gehalt an Dialkylfumarat. Diese Lagerung über mehrere Wochen führt zu erheblichen Logistik-Kosten innerhalb der Produktion. Ferner, obwohl das Produkt erst nach der Lagerung an den Kunden ausgeliefert wird, enthält es immer noch substanzielle Mengen Dialkylfumarat welches eine Sensibilisierung verursachen kann. So hergestellte Polyasparaginsäureester enthalten nach Beendigung der Reaktion üblicherweise noch Reste von 3 bis 20 Gew.-Prozent an nicht umgesetztem Fumar- und/oder Maleinsäureester.

Gemäß EP0403921 können Fumar- und/oder Maleinsäureester destillativ abgetrennt werden. Bedingungen unter denen die Abtrennung erfolgreich durchgeführt werden kann, nennt das Dokument nicht.
In DE102006002153A1 beschreibt ebenfalls die Herstellung von Asparaginsäureestern, die als Rohware, aber auch nach Lagerung noch mehrere Gew.-Prozent Fumarsäurediethylester enthalten. In Beispiel 3 wird erwähnt, dass der überschüssige Fumarsäurediethylester durch Dünnschichtdestillation vollständig entfernt wurde. Auch hier wird nicht auf die erforderlichen Destillationsbedingungen eingegangen. Auch wenn in diesem Dokument positive technische Effekte eines Gehalts von 2 bis 10 Gew.-Prozent Fumarsäurediethylester im Asparaginsäureester beschrieben werden, wie eine niedrige Viskosität bei gleichzeitig guter Topfzeit und Endhärte, sei an dieser Stelle darauf hingewiesen, dass ein so hoher Gehalt an Fumarsäurediethylester auch die oben bereits erwähnten nachteiligen Effekte hat.

Unter Berücksichtigung dieser Aspekte ist es wünschenswert, Polyasparaginsäureester mit weniger als 2 Gew.-Prozent an freien Fumarsäuredialkylestern bereitzustellen. Ein Gehalt von weniger als 0.01 Gew.-Prozent bringt hierbei keinen weiteren Vorteil bezüglich der Verarbeitungsbedingungen und lässt sich technisch nur mit erheblichem Aufwand realisieren.

Wie oben bereits erwähnt, kann der Fachmann dem Stand der Technik nicht entnehmen, wie eine Destillation zu erfolgen hat, um Polyasparaginsäureester mit einem niedrigen Gehalt von < 2 Gew.-Prozent Fumarsäurediethylester zu erhalten. Dies gilt insbesondere, wenn ein farbhelles Produkt erhalten werden soll, welches sich vorteilhaft in Beschichtungen einsetzen lässt.

Aufgrund der oben beschriebenen Nachteile ist die Aufgabe der vorliegenden Erfindung die Bereitstellung eines Verfahrens zur Reinigung von Polyasparaginsäureester-Zusammensetzungen zur Bereitstellung von Polyasparaginsäureestern, die sich mit minimalem Gesundheitsrisiko einfach in den gängigen Anwendungen applizieren lassen. Dieses Verfahren soll sich insbesondere für solche Polyasparaginsäureester eignen, die eine niedrige Platin-Cobalt-Farbzahl aufweisen.

Diese Aufgabe konnte gelöst werden durch das erfindungsgemäße Verfahren zur Reinigung von Polyasparaginsäureester-Zusammensetzungen zur Bereitstellung von gereinigten Polyasparaginsäureestern mit einem Gehalt von < 2 Gew.-Prozent Fumarsäuredialkylester.

Gegenstand der vorliegenden Erfindung ist ein Verfahren zur Reinigung von Polyasparaginsäureester-Zusammensetzungen enthaltend Polyasparaginsäureester der allgemeinen Formel (I)

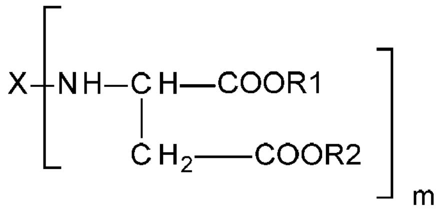

(I),

in welcher

X für einen m-wertigen, gegebenenfalls ein oder mehrere Heteroatomeenthaltenden organischen Rest steht, wie er durch Entfernung von primären Aminogruppen aus einem entsprechenden, aromatisch, (cyclo)aliphatisch bzw. araliphatisch gebundene primäre Aminogruppen aufweisenden Polyamin des Molekulargewichtsbereichs 60 bis 6000 g/mol erhalten werden kann, und der weitere, gegenüber Isocyanatgruppen reaktive und/oder bei Temperaturen bis 100°C inerte funktionelle Gruppen enthalten kann,

R1 und R2 für gleiche oder verschiedene organische Reste mit je 1 bis 18 Kohlenstoffatomen stehen,

m für eine ganze Zahl > 1 steht,

dadurch gekennzeichnet, dass der gereinigte Polyasparaginsäureester einen Anteil von 0.01 bis < 2 Gew.-Prozent Fumarsäuredialkylester enthält.

Ein bevorzugter Gegenstand der vorliegenden Erfindung ist das oben genannte Verfahren zur Reinigung von Polyasparaginsäureester-Zusammensetzungen der allgemeinen Formel (I), in welcher

X für einen m-wertigen, gegebenenfalls ein oder mehrere Heteroatome enthaltenden organischen Rest steht, wie er durch Entfernung von primären Aminogruppen aus Polyetherpolyaminen mit aliphatisch gebundenen primären Aminogruppen, 1,2 Diaminopropan, 1,4-Diaminobutan, 1,5-Diaminopentan, 1,6-Diaminohexan, 1,5-Diamino-2-methylpentan, 2,5 Diamino-2,5-dimethylhexan, 2,2,4- und/oder 2,4,4-Trimethyl-1,6-diaminohexan, 1,11-Diaminounodecan, 1,12-Diaminododecan, 1-Amino-3,3,5-trimehtyl-5-aminomethylcyclohexan, 2,4- und/oder 2,6-Hexahydrotoluylendiamin, 2,4'- und/oder 4,4'-Diaminodicyclohexylmethan oder 3,3'-Dimethyl-4, 4'-diamino-dicyclohexylmethan erhalten wird,

R1 und R2 für gleiche oder verschiedene Alkylreste mit je 1 bis 8 Kohlenstoffatomen stehen,

m für eine ganze Zahl > 1 steht,

dadurch gekennzeichnet, dass der gereinigte Polyasparaginsäureester einen Anteil von 0.01 - 0.2 Gew.-Prozent Fumarsäuredialkylester enthält.

Ein weiterer bevorzugter Gegenstand der vorliegenden Erfindung ist das oben genannte Verfahren zur Reinigung von Polyasparaginsäureester-Zusammensetzungen enthaltend Polyasparaginsäureester der allgemeinen Formel (I),
in welcher

X für einen m-wertigen organischen Rest steht, wie er durch Entfernung von primären Aminogruppen aus 3,3'-Dimethyl-4,4'-diamino-dicyclohexylmethan, 2,4'- und/oder 4,4'-Diaminodicyclohexylmethan, 1,5-Diamino-2-methylpentan erhalten werden kann,

R1 und R2 für gleiche oder verschiedene Alkylreste ausgewählt aus der Gruppe Methyl-, Ethyl-, Propyl-, iso-Propyl-, Butyl- oder iso-butyl-Reste stehen,

m für 2 steht,

dadurch gekennzeichnet, dass der gereinigte Polyasparaginsäureester einen Anteil von 0.01 - 0.2 Gew.-Prozent Fumarsäuredialkylester enthält.

Ein weiterer bevorzugter Gegenstand der vorliegenden Erfindung ist das oben genannte Verfahren zur Reinigung von Polyasparaginsäureester-Zusammensetzungen enthaltend Polyasparaginsäureester der allgemeinen Formel (I), in welcher

X für einen m-wertigen organischen Rest steht, wie er durch Entfernung von primären Aminogruppen aus 2,4' und/oder 4,4'-Diaminodicyclohexylmethan erhalten werden kann,
R1 und R2 für Ethyl-Reste stehen,
m für 2 steht,

dadurch gekennzeichnet, dass der gereinigte Polyasparaginsäureester einen Anteil von 0.01 - 0.1 Gew.-Prozent Fumarsäuredialkylester enthält.

Ein weiterer Gegenstand der vorliegenden Erfindung ist ein Verfahren zur Reinigung von Polyasparaginsäureester-Zusammensetzungen enthaltend Polyasparaginsäureester der allgemeinen Formel (I)

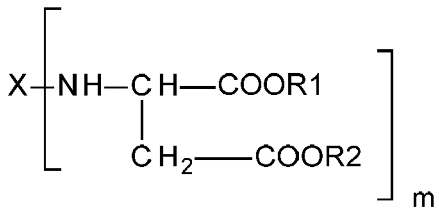

(I),

in welcher

X für einen m-wertigen, gegebenenfalls ein oder mehrere Heteroatome enthaltenden organischen Rest steht, wie er durch Entfernung von primären Aminogruppen aus einem entsprechenden, aromatisch, (cyclo)aliphatisch bzw. araliphatisch gebundene primäre Aminogruppen aufweisenden Polyamin des Molekulargewichtsbereichs 60 bis 6000 g/mol erhalten wird, und der weitere, gegenüber Isocyanatgruppen reaktive und/oder bei Temperaturen bis 100°C inerte funktionelle Gruppen enthalten kann,

R1 und R2 für gleiche oder verschiedene organische Reste mit je 1 bis 18 Kohlenstoffatomen stehen,

m für eine ganze Zahl > 1 und bevorzugt = 2 steht,

dadurch gekennzeichnet, dass substituierte Malein- und/oder Fumarsäureester der allgemeinen Formel (II)

R1OOC-C H=CH-COOR2 (II),

wobei die Reste R1 und R2 die oben genannten Bedeutungen haben,
mit primären Polyaminen der allgemeinen Formel (III)

$X\text{-}(\text{-}NH_2)_m$ (III),

wobei X und m die oben genannten Bedeutungen haben, zu einer Polyasparaginsäureester-Zusammensetzung enthaltend Polyasparaginsäureester der allgemeinen Formel (I) umgesetzt und in mindestens einem weiteren Schritt gereinigt werden, dadurch gekennzeichnet, dass der gereinigte Polyasparaginsäureester einen Anteil von 0.01 bis < 2 Gew.-Prozent Fumarsäuredialkylester enthält.

Ein bevorzugter Gegenstand der vorliegenden Erfindung ist das oben genannte Verfahren zur Reinigung von Polyasparaginsäureester-Zusammensetzungen der allgemeinen Formel (I), in welcher

X für einen m-wertigen, gegebenenfalls ein oder mehrere Heteroatome enthaltenden organischen Rest steht, wie er durch Entfernung von primären Aminogruppen aus Polyetherpolyaminen mit aliphatisch gebundenen primären Aminogruppen, 1,2 Diaminopropan, 1,4-Diaminobutan, 1,5,-Diaminopentan, 1,6-Diaminohexan, 1,5-Diamino-2-methylpentan, 2,5 Diamino-2,5-dimethylhexan, 2,2,4- und/oder 2,4,4-Trimethyl-1,6-diaminohexan, 1,11-Diaminounodecan, 1,12-Diaminododecan, 1-Amino-3,3,5-trimehtyl-5-aminomethylcyclohexan, 2,4- und/oder 2,6-Hexahydrotoluylendiamin, 2,4'- und/oder 4,4'-Diaminodicyclohexylmethan oder 3,3'-Dimethyl-4,4'-diamino-dicyclohexylmethan erhalten wird,
R1 und R2 für gleiche oder verschiedene Alkylreste mit je 1 bis 8 Kohlenstoffatomen stehen,
m für eine ganze Zahl > 1 und bevorzugt = 2 steht,

dadurch gekennzeichnet, dass substituierte Malein- und/oder Fumarsäureester der allgemeinen Formel (II), wobei die

Reste R1 und R2 die oben genannten Bedeutungen haben,
mit primären Polyaminen der allgemeinen Formel (III), wobei X und m die oben genannten Bedeutungen haben,
zu einer Polyasparaginsäureester-Zusammensetzung enthaltend Polyasparaginsäureester der allgemeinen Formel (I) umgesetzt, und in einem weiteren Schritt gereinigt werden, dadurch gekennzeichnet, dass der gereinigte Polyasparaginsäureester einen Anteil von 0.01 - 0.2 Gew.-Prozent Fumarsäuredialkylester enthält.

Ein weiterer bevorzugter Gegenstand der vorliegenden Erfindung ist das oben genannte Verfahren zur Reinigung von Polyasparaginsäureester-Zusammensetzungen enthaltend Polyasparaginsäureester der allgemeinen Formel (I), in welcher

| | |
|---|---|
| X | für einen m-wertigen organischen Rest steht, wie er durch Entfernung von primären Aminogruppen aus 3,3'-Dimethyl-4,4'-diamino-dicyclohexylmethan, 2,4- und/oder 4,4'-Diaminodicyclohexylmethan, 1,5-Diamino-2-methylpentan erhalten werden kann, |
| R1 und R2 | für gleiche oder verschiedene Alkylreste ausgewählt aus der Gruppe Methyl-, Ethyl-, Propyl-, iso-Propyl-, Butyl- oder iso-butyl-Reste stehen, |
| m | für 2 steht, |

dadurch gekennzeichnet, dass substituierte Malein- und/oder Fumarsäureester der allgemeinen Formel (II), wobei die Reste R1 und R2 die oben genannten Bedeutungen haben,
mit primären Polyaminen der allgemeinen Formel (III), wobei X und m die oben genannten Bedeutungen haben,
zu einer Polyasparaginsäureester-Zusammensetzung enthaltend Polyasparaginsäureester der allgemeinen Formel (I) umgesetzt und in einem weiteren Schritt gereinigt werden, dadurch gekennzeichnet, dass der gereinigte Polyasparaginsäureester einen Anteil von 0.01 - 0.2 Gew.-Prozent Fumarsäuredialkylester enthält.

Ein weiterer bevorzugter Gegenstand der vorliegenden Erfindung ist das oben genannte Verfahren zur Reinigung von Polyasparaginsäureester-Zusammensetzungen enthaltend Polyasparaginsäureester der allgemeinen Formel (I),
in welcher

| | |
|---|---|
| X | für einen m-wertigen organischen Rest steht, wie er durch Entfernung von primären Aminogruppen aus 2,4'- und/oder 4,4'-Diaminodicyclohexylmethan erhalten werden kann, |
| R1 und R2 | für Ethyl-Reste stehen, |
| m | für 2 steht, |

dadurch gekennzeichnet, dass substituierte Malein- und/oder Fumarsäureester der allgemeinen Formel (II), wobei die Reste R1 und R2 die oben genannten Bedeutungen haben,
mit primären Polyaminen der allgemeinen Formel (III), wobei X und m die oben genannten Bedeutungen haben,
zu einer Polyasparaginsäureester-Zusammensetzung enthaltend Polyasparaginsäureester der allgemeinen Formel (I) umgesetzt und in einem weiteren Schritt gereinigt werden, dadurch gekennzeichnet, dass der gereinigte Polyasparaginsäureester einen Anteil von 0.01 - 0.1 Gew.-Prozent Fumarsäuredialkylester enthält.

Vorzugsweise werden für das erfindungsgemäße Verfahren Malein- und/oder Fumarsäuredialkylester der Formel (II) eingesetzt, in denen für R1 und R2 für gleiche oder verschiedene Alkylreste mit je 1 bis 18 Kohlenstoffatomen, bevorzugt gleiche oder verschiedene Alkylreste mit je 1 bis 8 Kohlenstoffatomen und ganz besonders bevorzugt für jeweils Alkylreste wie Methyl-, Ethyl-, Propyl-, iso-Propyl-, Butyl- oder iso-butyl-Reste stehen. Besonders bevorzugt ist Maleinsäurediethylester. Beispielhaft und nicht abschließend seien für Verbindungen der allgemeinen Formel (II) folgende Diester genannt: Maleinsäure-dimethylester, -diethylester, -di-n-butylester, die entsprechenden Fumarsäureester oder die entsprechenden, in 2- und/oder 3-Stellung Methyl-substituierten Maleinsäure- oder Fumarsäureester.

Für das erfindungsgemäße Verfahren sind Polyamine der allgemeinen Formel (III) einsetzbar, ausgewählt aus der folgenden Gruppe: alle bekannten Polyamine mit primären Aminogruppen, die der allgemeinen Formel (III) entsprechen. Beispielhaft seien die folgenden Verbindungen genannt: Ethylendiamin, 1,2-Diaminopropan, 1,4-Diaminobutan, 1,5-Diaminopentan, 2,5-Diamino-2,5-dimethylhexan, 1,5-Diamino-2-methylpentan (Dytek®A, Fa DuPont), 1,6-Diaminohexan, 2,2,4-und/oder 2,4,4-Trimethyl-1,6-diaminohexan, 1,11-Diaminoundecan,1,12-Diaminododecan oder Triaminononan, Etheramine, wie z.B. 4,9-dioxadodecane-1,12-diamine, 4,7,10-trioxatridecane-1,13-diamine, oder höhermolekulare Polyetherpolyamine mit aliphatisch gebundenen, primären Aminogruppen, wie sie beispielsweise unter der Bezeichnung Jeffamin® von der Firma Huntsman vertrieben werden. Ebenfalls einsetzbar sind aliphatische polyzyklische Polyamine, wie Tricyclodecanbismethylamin (TCD-Diamin) oder Bis(aminomethyl)norbornane, aminofunktionelle Siloxane, z.B. Diaminopropylsiloxan G10 DAS (Fa. Momentive), Fettalkyl-basierte Amine, wie z.B. Fentamine der Fa. Solvay, Dimerfettsäurediamine wie z.B Priamine der Fa. Croda.

Bevorzugt werden im erfindungsgemäßen Verfahren Polyamine der allgemeinen Formel (III) eingesetzt, ausgewählt aus der Gruppe: Polyetherpolyaminen mit aliphatisch gebundenen primären Aminogruppen, 1,2 Diaminopropan,

1,4-Diaminobutan, 1,5-Diaminopentan, 1,6-Diaminohexan, 1,5-Diamino-2-methylpentan, 2,5 Diamino-2,5-dimethylhexan, 2,2,4- und/oder 2,4,4-Trimethyl-1,6-diaminohexan, 1,11-Diaminounodecan, 1,12-Diaminododecan, 1-Amino-3,3,5-trimehtyl-5-aminomethylcyclohexan, 2,4- und/oder 2,6-Hexahydrotoluylendiamin, 2,4'- und/oder 4,4'-Diamino-dicyclohexylmethan oder 3,3'-Dimethyl-4, 4'-diamino-dicyclohexylmethan.

Besonders bevorzugt werden im erfindungsgemäßen Verfahren Polyamine der allgemeinen Formel (III) eingesetzt ausgewählt aus der Gruppe: 3,3'-Dimethyl-4,4'-diamino-dicyclohexylmethan, 2,4-und/oder 4,4'-Diaminodicyclohexylmethan, 1,5-Diamino-2-methylpentan und ganz besonders bevorzugt ist die Verwendung von 2,4- und/oder 4,4'-Diaminodicyclohexylmethan.

Ebenfalls geeignet sind aromatische Polyamine wie beispielsweise 2,4- und/oder 2,6-Diaminotoluol oder 2,4'-und/oder 4,4'-Diaminodiphenylmethan oder deren mehrkernige Homologe. Ferner geeignet sind höhermolekulare Polyetherpolyamine mit aliphatisch gebundenen primären Aminogruppen, wie sie beispielsweise unter der Bezeichnung Jeffamine® vertrieben werden.

Vorzugsweise steht m für eine ganze Zahl >1 und bevorzugt für 2.

Die Herstellung der Polyasparaginsäureester-Zusammensetzung enthaltend Polyasparaginsäureester der allgemeinen Formel (I) erfolgt, indem die Verbindungen gemäß der allgemeinen Formel (II) und (III) bei Temperaturen zwischen 0°C und 100°C, vorzugsweise 20 bis 80°C und besonders bevorzugt 20 bis 60°C, in einem Verhältnis der Äquivalente der primären Aminogruppen der Verbindungen der allgemeinen Formel (III) zu den C=C-Doppelbindungsäquivalenten der Verbindung der allgemeinen Formel (II) von 1:10, vorzugsweise 1:5, ebenfalls bevorzugt 1:2 und besonders bevorzugt in einem Verhältnis von 1:1 umgesetzt werden. Danach kann die Polyasparaginsäureester-Zusammensetzung enthaltend Polyasparaginsäureester der allgemeinen Formel (I) für mehrere Wochen gelagert bzw. gereift werden und/oder durch das erfindungsgemäße Verfahren zur Reinigung aufgearbeitet werden.

Polyasparaginsäureester-Zusammensetzungen enthalten 70 bis 98 Gew.-Prozent, vorzugsweise 80 bis 98 Gew.-Prozent und besonders bevorzugt 85 bis 98 Gew.-Prozent Polyasparaginsäureester der allgemeinen Formel (I).

Im Rahmen der vorliegenden Erfindung ist der gereinigte Polyasparaginsäureester das Produkt, welches durch das erfindungsgemäße Verfahren zur Reinigung von Polyasparaginsäureester-Zusammensetzungen enthaltend Polyasparaginsäureester der allgemeinen Formel (I) erhalten wird. Es enthält vorzugsweise einen Anteil von 0.01 bis < 2 Gew.-Prozent, bevorzugt 0.01 - 0.2 Gew.-Prozent und besonders bevorzugt 0.01 - 0.1 Gew.-Prozent Fumarsäuredialkylester.

Bevorzugt sind im Rahmen der vorliegenden Erfindung gereinigte Polyasparaginsäureester, die eine Platin-Cobalt-Farbzahl ≤ 100, bevorzugt ≤ 50 und besonders bevorzugt ≤ 30 aufweisen und einen Anteil von 0.01 bis < 2 Gew.-Prozent, bevorzugt 0.01 - 0.2 Gew.-Prozent und besonders bevorzugt 0.01 - 0.1 Gew.-Prozent Fumarsäuredialkylester enthalten.

Ein weiterer Gegenstand der vorliegenden Erfindung ist das oben genannte Verfahren zur Reinigung, dadurch gekennzeichnet, dass Polyasparaginsäureester-Zusammensetzungen enthaltend Polyasparaginsäureester der allgemeinen Formel (I) mit einer Platin-Cobalt-Farbzahl ≤ 100 gereinigt werden.

Für eine möglichst vielseitige Anwendung der erfindungsgemäß gereinigten Polyasparaginsäureester für Beschichtungen ist es bevorzugt, wenn diese eine Platin-Cobalt-Farbzahl ≤ 100, besonders bevorzugt ≤ 50 aufweisen. Die Messung der Platin-Cobalt-Farbzahl erfolgt gemäß DIN EN ISO 6271:2016-05. Überraschenderweise ist es über das erfindungsgemäße Reinigungsverfahren möglich, eine Verfärbung der Polyasparaginsäureester der allgemeinen Formel (I) während der Reinigung weitgehend zu vermeiden. Eine Aufhellung bereits verfärbter Polyasparaginsäureester-Zusammensetzungen enthaltend Polyasparaginsäureester der allgemeinen Formel (I) ist hingegen nicht zu erwarten, so dass bevorzugt solche Polyasparaginsäureester-Zusammensetzungen enthaltend Polyasparaginsäureester der allgemeinen Formel (I) nach dem erfindungsgemäßen Verfahren gereinigt werden, die bereits eine Platin-Cobalt-Farbzahl ≤ 100, bevorzugt ≤ 50 und besonders bevorzugt ≤ 30 aufweisen.

Ein weiterer Gegenstand der vorliegenden Erfindung ist das oben genannte Verfahren zur Reinigung, wobei die Polyasparaginsäureester-Zusammensetzungen enthaltend Polyasparaginsäureester der allgemeinen Formel (I) durch Destillation gereinigt werden.

Ein weiterer Gegenstand der vorliegenden Erfindung ist das oben genannte Verfahren zur Reinigung, wobei die Polyasparaginsäureester-Zusammensetzungen enthaltend Polyasparaginsäureester der allgemeinen Formel (I) durch Destillation bei einem Druck unterhalb von 20 mbar und einer Temperatur unterhalb von 180 °C gereinigt werden.

Ein weiterer Gegenstand der vorliegenden Erfindung ist das oben genannte Verfahren zur Reinigung, wobei die Polyasparaginsäureester-Zusammensetzungen enthaltend Polyasparaginsäureester der allgemeinen Formel (I) durch Destillation gereinigt wird, dadurch gekennzeichnet, dass

a) die Destillation bei einem Druck zwischen 0.005 und 2 mbar erfolgt und
b) die Temperatur des Sumpfablaufs beim Austritt aus der Destillationsapparatur ≤ 170 °C und ≥ der Temperatur liegt, die sich aus der folgenden Formel (IV) ergibt:

$$T(Sumpfablauf) = 27 \times \ln(p) + 150 \qquad (IV),$$

wobei T(Sumpfablauf) für die Temperatur des Sumpfablaufs in °C und
p für den Druck in der Destillationsapparatur in mbar stehen.

Vorzugsweise erfolgt die Destillation bei einem Druck zwischen 0.005 und 2 mbar, bevorzugt zwischen 0.005 und 0.8 mbar, besonders bevorzugt zwischen 0.005 und 0.2 mbar.

Durch Einhalten dieses Druckbereichs wird gewährleistet, dass einerseits moderate Temperaturen im Sumpfablauf ausreichen, um die Fumarsäuredialkylester im gewünschten Umfang abzureichern, andererseits das Verfahren im technischen Maßstab anwendbar bleibt. Bei geringerem Druck wird die Gasdichte zu gering und die erforderlichen Apparate deshalb so groß, dass das Verfahren aus wirtschaftlicher Sicht nachteilig wird.

Bevorzugt ist die Temperatur des Sumpfablaufs ≤ 170 °C, dabei jedoch mindestens 20 K oberhalb der Temperatur, die sich aus der Formel (IV) ergibt, besonders bevorzugt liegt sie zwischen 20 K und 40 K oberhalb der Temperatur, die sich aus der Formel (IV) ergibt jedoch nicht oberhalb von 170 °C.

Ein weiterer Gegenstand der vorliegenden Erfindung ist das oben genannte Verfahren zur Reinigung, dadurch gekennzeichnet, dass die Zeit während der die Polyasparaginsäureester-Zusammensetzung enthaltend Polyasparaginsäureester der allgemeinen Formel (I), Temperaturen oberhalb von 100 °C ausgesetzt ist, weniger als 2 Stunden beträgt.

In einer weiteren bevorzugten Ausführungsform wird deshalb die Zeit während der die Polyasparaginsäureester-Zusammensetzungen enthaltend Polyasparaginsäureester der allgemeinen Formel (I) Temperaturen oberhalb von 100 °C ausgesetzt sind auf weniger als 2 Stunden, bevorzugt weniger als 30 Minuten, besonders bevorzugt weniger als 10 Minuten begrenzt. Je höher die Temperatur ist, desto kürzer sollte die Verweilzeit bei dieser Temperatur gewählt werden. Dies kann zum Beispiel durch eine geeignete Dimensionierung der Apparate und Rohrleitungen der Destillationsapparatur erreicht werden und es hat den Vorteil, dass die thermische Belastung des Materials und eine damit einhergehende Verfärbung möglichst gering gehalten werden.

Überraschend wurde gefunden, dass eine längere Verweilzeit bei erhöhter Temperatur nicht nur zu einer Erhöhung der Farbzahl führt, sondern auch die Rückspaltung des Polyasparaginsäureesters zur Folge hat. Diese Rückspaltung führt dazu, dass neue Fumarsäuredialkylester entstehen. Der naheliegende Versuch, die Fumarsäuredialkylester im Produkt bis zur gewünschten Spezifikation weiter abzureichern, indem Temperatur und/oder Verweilzeit erhöht werden, gelingt nicht. Dies wirkt sich insbesondere nach dem Austritt aus der Destillationsapparatur aus, da dann neu gebildete Fumarsäuredialkylester nicht mehr entfernt werden sondern im gereinigten Produkt verbleiben.

Ein weiterer Gegenstand er vorliegenden Erfindung ist das oben genannte Verfahren zur Reinigung, dadurch gekennzeichnet, dass der Sumpfablauf enthaltend den gereinigten Polyasparaginsäureester direkt nach Verlassen der Destillationsapparatur auf Temperaturen unterhalb von 100 °C abgekühlt wird.

Ein weiterer Gegenstand er vorliegenden Erfindung ist das oben genannte Verfahren zur Reinigung, dadurch gekennzeichnet, dass der Sumpfablauf enthaltend den gereinigten Polyasparaginsäureester direkt nach Verlassen der Destillationsapparatur auf Temperaturen unterhalb von 100 °C abgekühlt wird, wobei die mittlere Verweilzeit des Sumpfablaufs zwischen Austritt aus der Destillationsapparatur und Eintritt in einen Kühler unterhalb von 5 Minuten beträgt.

Vorzugsweise wird der Sumpfablauf deshalb direkt nach Verlassen der Destillationsapparatur auf Temperaturen unterhalb von 100 °C, bevorzugt unterhalb von 80 °C abgekühlt. Vorliegend wird unter "direkt nach Verlassen der Destillationsapparatur" verstanden, dass die mittlere Verweilzeit des Sumpfablaufs zwischen Austritt aus der Destillationsapparatur und Eintritt in einen Kühler unterhalb von 5 Minuten, bevorzugt unterhalb von 2 Minuten und besonders bevorzugt unterhalb von 1 Minute liegt.

Bei einer mehrstufigen Destillation ist es bevorzugt, wenn mindestens die letzte Stufe der Destillation durch die oben angegebenen Bedingungen gekennzeichnet ist.

Geeignete Apparate für die erfindungsgemäße Destillation sind beispielsweise Fallfilmverdampfer, Dünnschichtverdampfer und/oder Kurzwegverdampfer. Bevorzugt ist die Verwendung von Dünnschichtverdampfern und/oder Kurzwegverdampfern, da diese eine besonders schonende Destillation ermöglichen.

Ein weiterer Gegenstand er vorliegenden Erfindung ist das oben genannte Verfahren zur Reinigung, dadurch gekennzeichnet, dass die Destillation unter Verwendung von Fallfilmverdampfer, Dünnschichtverdampfer und/oder Kurzwegverdampfer durchgeführt wird.

Ein weiterer Gegenstand er vorliegenden Erfindung ist das oben genannte Verfahren zur Reinigung, dadurch gekennzeichnet, dass die Destillation zweistufig unter Verwendung einer Kombination aus Dünnschichtverdampfer und anschließendem Kurzwegverdampfer durchgeführt wird.

In einer weiteren bevorzugten Ausführungsform des erfindungsgemäßen Verfahrens zur Reinigung erfolgt die Destillation zweistufig, das heißt in zwei in Serie verschalteten Verdampfern wobei der gereinigte Polyasparaginsäureester jeweils am Sumpf der Verdampfer anfällt. Um auch im technisch relevanten Maßstab die Druckbedingungen für

das erfindungsgemäße Verfahren zur Reinigung einzuhalten ist es dabei besonders bevorzugt, eine Kombination aus Dünnschichtverdampfer und anschließendem Kurzwegverdampfer zu wählen. Mit Hilfe einer solchen Anordnung können die Druckverluste minimiert werden und der Polyasparaginsäureester in der zweiten Stufe bei besonders niedrigem Druck, beispielsweise zwischen 0.005 und 0.2 mbar und damit auch bei niedrigen Temperaturen schonend destilliert werden.

Ein weiterer Gegenstand der vorliegenden Erfindung sind Polyasparaginsäureester-Zusammensetzungen enthaltend Verbindungen der allgemeinen Formel (I)

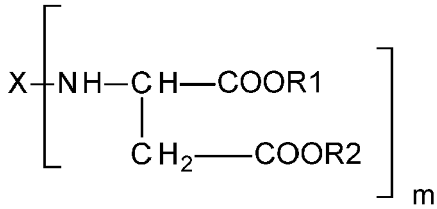

(I),

in welcher

X für einen m-wertigen, gegebenenfalls ein oder mehrere Heteroatomeenthaltenden organischen Rest steht, wie er durch Entfernung von primären Aminogruppen aus einem entsprechenden, aromatisch, (cyclo)aliphatisch bzw. araliphatisch gebundene primäre Aminogruppen aufweisenden Polyamin des Molekulargewichtsbereichs 60 bis 6000 g/mol erhalten wird, und der weitere, gegenüber Isocyanatgruppen reaktive und/oder bei Temperaturen bis 100°C inerte funktionelle Gruppen enthalten kann,

R1 und R2 für gleiche oder verschiedene organische Reste mit je 1 bis 18 Kohlenstoffatomen stehen,

m für eine ganze Zahl > 1 und bevorzugt = 2 steht,

dadurch gekennzeichnet, dass ein Anteil von 0.01 bis < 2 Gew.-Prozent Fumarsäuredialkylester enthalten ist und eine Platin-Cobalt-Farbzahl ≤ 100 vorliegt.

Ein weiterer Gegenstand der vorliegenden Erfindung sind Polyasparaginsäureester-Zusammensetzungen enthaltend Verbindungen der allgemeinen Formel (I), in welcher

X für einen m-wertigen, gegebenenfalls ein oder mehrere Heteroatome enthaltenden organischen Rest steht, wie er durch Entfernung von primären Aminogruppen aus Polyetherpolyaminen mit aliphatisch gebundenen primären Aminogruppen, 1,2 Diaminopropan, 1,4-Diaminobutan, 1,5-Diaminopentan, 1,6-Diaminohexan, 1,5-Diamino-2-methylpentan, 2,5 Diamino-2,5-dimethylhexan, 2,2,4- und/oder 2,4,4-Trimethyl-1,6-diaminohexan, 1,11-Diaminounodecan, 1,12-Diaminododecan, 1-Amino-3,3,5-trimehtyl-5-aminomethylcyclohexan, 2,4- und/oder 2,6-Hexahydrotoluylendiamin, 2,4'- und/oder 4,4'-Diamino-dicyclohexylmethan oder 3,3'-Dimethyl-4, 4'-diamino-dicyclohexylmethan erhalten wird,

R1 und R2 für gleiche oder verschiedene Alkylreste mit je 1 bis 8 Kohlenstoffatomen stehen,

m für eine ganze Zahl > 1 und bevorzugt = 2 steht,

dadurch gekennzeichnet, dass ein Anteil von 0.01 - 0.2 Gew.-Prozent Fumarsäuredialkylester enthalten ist und eine Platin-Cobalt-Farbzahl ≤ 50 vorliegt.

Ein weiterer Gegenstand der vorliegenden Erfindung sind Polyasparaginsäureester-Zusammensetzungen enthaltend Verbindungen der allgemeinen Formel (I), in welcher

X für einen m-wertigen organischen Rest steht, wie er durch Entfernung von primären Aminogruppen aus 3,3'-Dimethyl-4,4'-diamino-dicyclohexylmethan, 2,4- und/oder 4,4'-Diaminodicyclohexylmethan, 1,5-Diamino-2-methylpentan erhalten werden kann,

R1 und R2 für gleiche oder verschiedene Alkylreste ausgewählt aus der Gruppe Methyl-, Ethyl-, Propyl-, iso-Propyl-, Butyl- oder iso-butyl-Reste stehen,

m für 2 steht,

dadurch gekennzeichnet, dass ein Anteil von 0.01 - 0.2 Gew.-Prozent Fumarsäuredialkylester enthalten ist und eine Platin-Cobalt-Farbzahl ≤ 30 vorliegt.

Ein weiterer Gegenstand der vorliegenden Erfindung sind Polyasparaginsäureester-Zusammensetzungen enthaltend Verbindungen der allgemeinen Formel (I), in welcher

| | |
|---|---|
| X | für einen m-wertigen organischen Rest steht, wie er durch Entfernung von primären Aminogruppen aus 2,4- und/oder 4,4'-Diaminodicyclohexylmethan erhalten werden kann, |
| R1 und R2 | für Ethyl-Reste stehen, |
| m | für 2 steht, |

dadurch gekennzeichnet, dass ein Anteil von 0.01 - 0.1 Gew.-Prozent Fumarsäuredialkylester enthalten ist und eine Platin-Cobalt-Farbzahl ≤ 30 vorliegt.

## Experimenteller Teil

Im Folgenden wird die Erfindung anhand von Beispielen erläutert.

Als Ausgangssubstanz diente Tetraethyl-N,N'-(methylendicyclohexan-4,1-diyl)bis-DL-aspartat (CAS-Nr. 136210-30-5) welches eine Platin-Cobalt-Farbzahl von 15 und einen Gehalt an Fumarsäurediethylester von ca. 5 Gew.-Prozent aufwies. Im Folgenden wird dieser als Polyasparaginsäureester A bezeichnet.

Die quantitative Bestimmung des Gehalts an Fumarsäurediethylester in den Proben erfolgte mittels einer GC-Methode mit internem Standard. Es wurde ein GC Agilent mit einer fused silica Kapillare und einem FID Detektor verwendet. Die Temperatur des Injektors (Splitausgang) war 180°C, als Trägergas wurde Helium verwendet. Die Bestimmungsgrenze dieser Methode betrug 300 ppm.

Beispiel 1:

Polyasparaginsäureester A wurde zunächst durch Destillation aufgereinigt, so dass der Gehalt an Fumarsäuredialkylester (FADE) im gereinigten Polyasparaginsäureester 0.07 Gew.-Prozent betrug. Proben dieses gereinigten Polyasparaginsäureesters wurden anschließend bei 70 °C, bei 100 °C und bei 130 °C getempert. Zu Beginn sowie nach 2 h und nach 5 h wurden die getemperten Proben auf ihren Gehalt an FADE hin untersucht. Die Ergebnisse der Temperung sind in der folgenden Tabelle 1 zusammengefasst. Die Prozentangaben bedeuten jeweils Gewichtsprozent an FADE in der Probe. Tabelle 1:

| Temperatur | Startwert | 2h Temperung | 5 h Temperung |
|---|---|---|---|
| 70 °C | 0.070% | 0.065% | 0.075% |
| 100 °C | 0.050% | 0.095% | 0.150% |
| 130 °C | 0.070% | 0.260% | 0.75% |

**Beispiel 2**

200 kg des Polyasparaginsäureesters A werden bei 0.25 mbar in einem Dünnschichtverdampfer destilliert. Die Temperatur des Sumpfablaufs beträgt dabei 140 °C und die mittlere Verweilzeit des Produkts im Verdamfer ca. 5 Minuten. Das destillierte Produkt wird ohne Durchlaufen eines weiteren Abkühlschritts in einem Stahlbehälter aufgefangen und dort zwischengelagert. Dabei kühlt es langsam ab. Nach 2 Stunden fällt die Temperatur auf unter 100 °C ab. Es wird eine Probe entnommen und auf den Gehalt an FADE hin untersucht. Dieser beträgt 0.22 Gew.-Prozent und die Platin-Cobalt-Farbzahl der Probe beträgt 33.

**Beispiel 3**

200 kg des Polyasparaginsäureesters A werden wie in Beispiel 2 destilliert. Nun wird jedoch der Sumpfablauf direkt nach dem Verlassen des Verdampfers in einen Wärmetauscher geleitet und dort auf 70 °C abgekühlt, bevor das Produkt zur Lagerung in einen Stahlbehälter abgefüllt wird. Nach 2 Stunden wird eine Probe auf ihren Gehalt an FADE hin untersucht. Dieser beträgt 0.07 Gew.-Prozent und die Platin-Cobalt-Farbzahl der Probe beträgt 30.

**Beispiel 4**

200 kg des Polyasparaginsäureesters A werden wie in Beispiel 3 destilliert, mit dem Unterschied, dass diesmal

ein Druck von 1 mbar eingestellt wird. Eine Probe des eingelagerten Produkts wird nach 2 Stunden auf ihren Gehalt an FADE hin untersucht. Dieser beträgt 0.7 Gew.-Prozent und die Platin-Cobalt-Farbzahl der Probe beträgt 28.

**Beispiel 5**

200 kg des Polyasparaginsäureesters A werden wie in Beispiel 2 destilliert, also ohne rasche Abkühlung des Sumpfablaufs. Im Unterschied zu Beispiel 2 wird diesmal ein Druck von 0.5 mbar eingestellt und die Heizleistung erhöht, so dass die Temperatur des Sumpfablaufs 175 °C beträgt. Die Probe nach 2 h hat einen Gehalt an FADE von 0.37 Gew.-Prozent. Außerdem zeigt sich eine Verfärbung des Produkts. Die Platin-Cobalt-Farbzahl beträgt 240.

## Patentansprüche

1. Verfahren zur Reinigung von Polyasparaginsäureester-Zusammensetzungen enthaltend Polyasparaginsäureester der allgemeinen Formel (I)

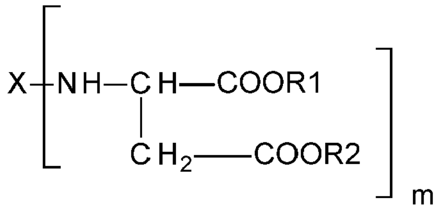

(I),

in welcher

X für einen m-wertigen, gegebenenfalls ein oder mehrere Heteroatome enthaltenden organischen Rest steht, wie er durch Entfernung von primären Aminogruppen aus einem entsprechenden, aromatisch, (cyclo)aliphatisch bzw. araliphatisch gebundene primäre Aminogruppen aufweisenden Polyamin des Molekulargewichtsbereichs 60 bis 6000 g/mol erhalten werden kann, und der weitere, gegenüber Isocyanatgruppen reaktive und/oder bei Temperaturen bis 100°C inerte funktionelle Gruppen enthalten kann,
R1 und R2 für gleiche oder verschiedene organische Reste mit je 1 bis 18 Kohlenstoffatomen stehen,
m für eine ganze Zahl > 1 und bevorzugt = 2 steht,

**dadurch gekennzeichnet, dass** der gereinigte Polyasparaginsäureester einen Anteil von 0.01 bis < 2 Gew.-Prozent Fumarsäuredialkylester enthält.

2. Verfahren zur Reinigung von Polyasparaginsäureester-Zusammensetzungen enthaltend Polyasparaginsäureester der allgemeinen Formel (I)

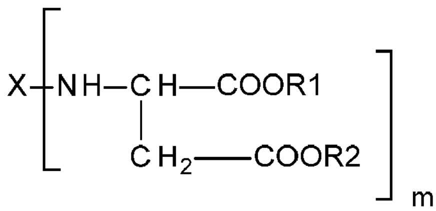

(I),

in welcher

X für einen m-wertigen, gegebenenfalls ein oder mehrere Heteroatome enthaltenden organischen Rest steht, wie er durch Entfernung von primären Aminogruppen aus einem entsprechenden, aromatisch, (cyclo)aliphatisch bzw. araliphatisch gebundene primäre Aminogruppen aufweisenden Polyamin des Molekulargewichtsbereichs 60 bis 6000 g/mol erhalten werden kann, und der weitere, gegenüber Isocyanatgruppen reaktive und/oder bei Temperaturen bis 100°C inerte funktionelle Gruppen enthalten kann,
R1 und R2 für gleiche oder verschiedene organische Reste mit je 1 bis 18 Kohlenstoffatomen stehen,

m für eine ganze Zahl > 1 und bevorzugt = 2 steht,

**dadurch gekennzeichnet, dass** substituierte Malein- oder Fumarsäureester der allgemeinen Formel (II)

R1OOC -C H=C H-COOR2 (II),

wobei die Reste R1 und R2 die oben genannten Bedeutungen haben,
mit primären Polyaminen der allgemeinen Formel (III)

$X\text{-}(\text{-}NH_2)_m$ (III),

wobei X und m die oben genannten Bedeutungen haben,
zu einer Polyasparaginsäureester-Zusammensetzung der allgemeinen Formel (I) umgesetzt und in einem weiteren Schritt gereinigt werden, **dadurch gekennzeichnet, dass** der gereinigte Polyasparaginsäureester einen Anteil von 0.01 bis < 2 Gew.-Prozent Fumarsäuredialkylester enthält.

**3.** Verfahren gemäß Ansprüchen 1 und 2, wobei die Polyasparaginsäureester-Zusammensetzung enthaltend Polyasparaginsäureester der allgemeinen Formel (I) durch Destillation gereinigt wird.

**4.** Verfahren gemäß Anspruch 3, wobei die Destillation bei einem Druck unterhalb von 20 mbar und einer Temperatur unterhalb von 180 °C erfolgt.

**5.** Verfahren gemäß Ansprüchen 3 und 4, wobei die Polyasparaginsäureester-Zusammensetzung enthaltend Polyasparaginsäureester der allgemeinen Formel (I) durch Destillation gereinigt wird, **dadurch gekennzeichnet, dass**

a) die Destillation bei einem Druck zwischen 0.005 und 2 mbar erfolgt und
b) die Temperatur des Sumpfablaufs beim Austritt aus der Destillationsapparatur ≤ 170 °C und ≥ der Temperatur liegt, die sich aus der folgenden Formel (IV) ergibt:

$$T(\text{Sumpfablauf}) = 27 \times \ln(p) + 150 \qquad (IV),$$

wobei

T(Sumpfablauf) für die Temperatur des Sumpfablaufs in °C und
p für den Druck in der Destillationsapparatur in mbar stehen.

**6.** Verfahren gemäß Ansprüchen 1 bis 5, **dadurch gekennzeichnet, dass** die Zeit während der der Polyasparaginsäureester enthaltend Polyasparaginsäureester der allgemeinen Formel (I), Temperaturen oberhalb von 100 °C ausgesetzt ist, weniger als 2 Stunden beträgt.

**7.** Verfahren gemäß Ansprüchen 3 bis 6, **dadurch gekennzeichnet, dass** der Sumpfablauf enthaltend den gereinigten Polyasparaginsäureester direkt nach Verlassen der Destillationsapparatur auf Temperaturen unterhalb von 100 °C abgekühlt wird.

**8.** Verfahren gemäß Ansprüchen 3 bis 7, **dadurch gekennzeichnet, dass** der Sumpfablauf enthaltend den gereinigten Polyasparaginsäureester direkt nach Verlassen der Destillationsapparatur auf Temperaturen unterhalb von 100 °C abgekühlt wird, wobei die mittlere Verweilzeit des Sumpfablaufs zwischen Austritt aus der Destillationsapparatur und Eintritt in einen Kühler unterhalb von 5 Minuten beträgt.

**9.** Verfahren gemäß Ansprüchen 3 bis 8, **dadurch gekennzeichnet, dass** die Destillation unter Verwendung von Fallfilmverdampfer, Dünnschichtverdampfer und/oder Kurzwegverdampfer durchgeführt wird.

**10.** Verfahren gemäß Ansprüchen 3 bis 9, **dadurch gekennzeichnet, dass** die Destillation zweistufig unter Verwendung einer Kombination aus Dünnschichtverdampfer und anschließendem Kurzwegverdampfer durchgeführt wird.

**11.** Verfahren gemäß Ansprüchen 1 bis 10, **dadurch gekennzeichnet, dass** Polyasparaginsäureester-Zusammensetzung enthaltend Polyasparaginsäureester der allgemeinen Formel (I) mit einer Platin-Cobalt-Farbzahl ≤ 100 gereinigt

werden.

**12.** Polyasparaginsäureester-Zusammensetzungen enthaltend Verbindungen der allgemeinen Formel (I)

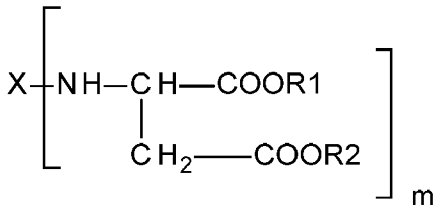

(I),

in welcher

X für einen m-wertigen, gegebenenfalls ein oder mehrere Heteroatome enthaltenden organischen Rest steht, wie er durch Entfernung von primären Aminogruppen aus einem entsprechenden, aromatisch, (cyclo)aliphatisch bzw. araliphatisch gebundene primäre Aminogruppen aufweisenden Polyamin des Molekulargewichtsbereichs 60 bis 6000 g/mol erhalten wird, und der weitere, gegenüber Isocyanatgruppen reaktive und/oder bei Temperaturen bis 100°C inerte funktionelle Gruppen enthalten kann,
R1 und R2 für gleiche oder verschiedene organische Reste mit je 1 bis 18 Kohlenstoffatomen stehen,
m für eine ganze Zahl > 1 und bevorzugt = 2 steht,

**dadurch gekennzeichnet, dass** ein Anteil von 0.01 bis < 2 Gew.-Prozent Fumarsäuredialkylester enthalten ist und eine Platin-Cobalt-Farbzahl ≤ 100 vorliegt.

Europäisches Patentamt
European Patent Office
Office européen des brevets

# EUROPÄISCHER RECHERCHENBERICHT

Nummer der Anmeldung
EP 17 19 1793

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | DE 10 2006 002153 A1 (BAYER MATERIALSCIENCE AG [DE]) 19. Juli 2007 (2007-07-19) * Absatz [0038]; Anspruch 1 * ----- | 1-12 | INV. C07C227/40 C07C229/46 |
| X | EP 0 816 326 A1 (BAYER AG [DE]) 7. Januar 1998 (1998-01-07) * Beispiel 7 * ----- | 12 | |

| | RECHERCHIERTE SACHGEBIETE (IPC) |
|---|---|
| | C07C |

Der vorliegende Recherchenbericht wurde für alle Patentansprüche erstellt

1

| Recherchenort | Abschlußdatum der Recherche | Prüfer |
|---|---|---|
| München | 9. März 2018 | Kleidernigg, Oliver |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**ANHANG ZUM EUROPÄISCHEN RECHERCHENBERICHT ÜBER DIE EUROPÄISCHE PATENTANMELDUNG NR.** EP 17 19 1793

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

09-03-2018

| Im Recherchenbericht angeführtes Patentdokument | Datum der Veröffentlichung | Mitglied(er) der Patentfamilie | Datum der Veröffentlichung |
|---|---|---|---|
| DE 102006002153 A1 | 19-07-2007 | KEINE | |
| EP 0816326 A1 | 07-01-1998 | AT 192734 T | 15-05-2000 |
| | | CA 2209131 A1 | 02-01-1998 |
| | | EP 0816326 A1 | 07-01-1998 |
| | | ES 2148877 T3 | 16-10-2000 |
| | | JP 4173567 B2 | 29-10-2008 |
| | | JP H1087583 A | 07-04-1998 |
| | | US 5821326 A | 13-10-1998 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 0403921 A
- EP 0639628 A
- EP 0667362 A
- EP 0689881 A
- US 5214086 A
- US 6605684 B
- EP 0573860 A
- EP 0699696 A
- EP 0596360 A
- EP 0893458 A
- DE 19701835
- DE 102006002153
- EP 1767559 A
- WO 2001007504 A
- WO 2001007399 A
- WO 2004033517 A
- US 6458293 B
- EP 1426397 A
- US 5243012 A
- DE 102006002153 A1


### In der Beschreibung aufgeführte Nicht-Patentliteratur


- **HAUBEN WEYL.** *Meth. d. Org. Chemie Bd.,* 1957, vol. 11/1, 272
- *Usp. Khim.,* 1969, vol. 38, 1933